# EUROPÄISCHE PATENTSCHRIFT

(11) **EP 1 154 703 B1**
(45) Veröffentlichungstag und Bekanntmachung des Hinweises auf die Patenterteilung: **02.05.2003**
(21) Anmeldenummer: 00920442.1
(22) Anmeldetag: 23.02.2000
(51) Int. Cl.: A23J 3/34

(54) **VERFAHREN ZUR HERSTELLUNG VON EIWEISSHYDROLYSATEN**
METHOD FOR PREPARING PROTEIN HYDROLYSATES
PROCEDE DE PREPARATION D'HYDROLYSATS DE PROTEINES

(30) Priorität: 23.02.1999 DE 19907726
(43) Veröffentlichungstag der Anmeldung: 21.11.2001
(73) Patentinhaber: NEUMÜLLER, Waldemar, D-37077 Göttingen (DE)
(72) Erfinder: NEUMÜLLER, Waldemar, D-37077 Göttingen (DE)
(74) Vertreter: Patentanwälte Rehberg + Hüppe
(86) Internationale Anmeldenummer: EP0001484
(87) Internationale Veröffentlichungsnummer: WO00049886

(56) Entgegenhaltungen:
- EP-A- 0 087 246
- WO-A-95/14394
- US-A- 3 857 966

## Beschreibung

Die Erfindung bezieht sich auf ein Verfahren zur Herstellung von Eiweißhydrolysaten aus einer Eiweiße in konzentrierter oder isolierter Form enthaltenden Ausgangssubstanz.

Eiweißhydrolysate sind Eiweißfragmente unterschiedlicher Länge, die durch chemische Hydrolyse mittels Alkali oder Säure oder durch enzymatische Hydrolyse mit Proteasen entstehen können. Bei der chemischen Hydrolyse können als Nachteile Proteinschäden wie Lysinoalanin und Chlorpropanol entstehen. Bekannte Nachteile der enzymatischen Hydrolyse sind lange Hydrolysezeit und Bitterpeptide. Zudem sind bei der enzymatischen Hydrolyse die Ausbeuten an Eiweißhydrolysaten bezogen auf die eingesetzten Eiweiße deutlich geringer als bei der chemischen Hydrolyse. Demgegenüber verläuft die chemische Hydrolyse weitgehend unspezifisch. Bei Abwägung aller Faktoren ist derzeit die enzymatische Hydrolyse bei der Herstellung von Eiweißhydrolysaten für den Nahrungsmittel- und Pharmabereich bevorzugt.

Verfahren zur Herstellung von Eiweißhydrolysaten aus einer Eiweiße in konzentrierter oder isolierter Form enthaltenden Ausgangssubstanz unter enzymatischer Hydrolyse nach dem Stand der Technik weisen grundsätzlich einen ähnlichen Aufbau auf, wobei die Eiweiße vor oder nach der enzymatischen Hydrolyse in der Regel bei einem alkalischen pH-Wert gelöst werden.

So ist es aus der US 1 231 652 zur Verringerung der Viskosität von Eiweißdispersionen bekannt, eine enzymatische Hydrolyse am isoelektrischen Punkt der Eiweiße mit sauren Proteasen durchzuführen, deren Aktivität in diesem pH-Bereich liegt. Anschließend wird der pH-Wert mittels Alkali teilweise bis auf 11,8 angehoben, und bei Temperaturen von 50-85°C werden die Eiweiße für 1 bis 2 Stunden alkalisch hydrolysiert. Dabei erfolgt eine Lösung der Eiweiße, die dann nochmals mittels der Protease Pepsin im stark sauren pH-Bereich gespalten werden. Bei diesem bekannten Verfahren ist von Nachteil, daß mit dem ständig wechselnden pH-Wert der die Eiweiße enthaltenden Dispersion bzw. Lösung eine starke Salzbildung verbunden ist und daß die enzymatische Hydrolyse nur im sauren pH-Wert durchgeführt wird, wobei Hydrolysezeiten von mehr als 20 Stunden erforderlich sind, um eine annehmbare Eiweißhydrolysatausbeute zu erzielen.

Aus der US 4 473 589 ist es bekannt, eine enzymatische Hydrolyse mit einer alkalischen Hydrolyse zu verbinden. Dazu wird unter stark alkalischen Bedingungen zunächst bei pH-Werten über 12 alkalisch hydrolysiert, und dann wird die Eiweißdispersion mittels einer alkalischen Protease weiter verflüssigt. Die Temperaturen liegen dabei für die alkalische Hydrolyse über 1 bis 2 Stunden bei 50 bis 80°C und bei der enzymatischen Hydrolyse bei 40 bis 60°C. Die Dauer der enzymatischen Hydrolyse beträgt bis zu 48 Stunden. Das Verfahren nach der US 4 473 589 führt wie auch das zuletzt beschriebene Verfahren nach der US 1 231 652 durch die hohen Temperaturen bei gleichzeitig hohen pH-Werten zu ausgeprägten Alkalischäden der erhaltenden Eiweißhydrolysate. Dies schränkt eine Verwendung der erhaltenen Eiweißhydrolysate für den Nahrungsmittel- und Pharmabereich stark ein. Zudem sind gerade die langen Hydrolysezeiten bei dem Verfahren nach der US 4 473 589 für eine wirtschaftliche Herstellung von Eiweißhydrolysaten in breiter Anwendung ungeeignet.

Ein Verfahren zur Herstellung eines Soja-Eiweißhydrolysats ist aus der EP 0 199 981 A2 bekannt. Dabei wird eine neutrale Protease im neutralen pH-Bereich eingesetzt. Um den Angriff dieser neutralen Protease zu unterstützen, wird zunächst eine feine Dispersion der Ausgangssubstanz dadurch hergestellt, daß diese bei 60 bis 100°C über einen Druckabfall von 100 bis 800 bar homogenisiert wird, so daß die Ausgangssubstanz mit kleiner Partikelgröße und damit großer Angriffsfläche vorliegt. Je nach der erwünschten Löslichkeit des angestrebten Produkts wird dann für eine Dauer von 5 bis 180 min. hydrolysiert. Ohne weitere Aufarbeitung wird die sich ergebende Dispersion nach Inaktivierung der eingesetzten Enzyme getrocknet. Zu den Nachteilen dieses bekannten Verfahrens gehört, daß man eine Dispersion aus verschiedensten Eiweißfragmenten bis hin zu nicht umgesetzten Eiweißen erhält. Außerdem ist die Verfügbarkeit neutraler Proteasen begrenzt. Weiterhin führen die hohen Drücke bei den gleichzeitig hohen Temperaturen während der Homogenisation zu starken Proteindenaturierungen, so daß hierdurch die Angriffsmöglichkeiten der Protease stark eingeschränkt werden, was die Hydrolysezeiten verlängert. Insgesamt bleiben die erhaltenen Eiweißhydrolysate auf wenige Anwendungsbereiche beschränkt.

Aus der EP 0 298 419 A2 ist ein Verfahren zur Herstellung von Eiweißhydrolysaten aus einer Eiweiß in konzentrierter oder isolierter Form enthaltenden Ausgangssubstanz bekannt, das der vorliegenden Erfindung von allem vorliegenden Stand der Technik am nächsten kommt. Bei diesem bekannten Verfahren werden pflanzliche Eiweiße über 10 bis 600 min. chemisch mit Säure oder Alkali bei 60 bis 180°C und/oder enzymatisch und/oder mit reduzierenden oder oxidierenden Mitteln hydrolysiert. Die Art und Weise der Hydrolyse soll dabei durch die Anwendung des herzustellenden Eiweißhydrolysats bestimmt werden. Ein klares Konzept für die Herstellung von Eiweißhydrolysaten ergibt sich aus der EP 0 298 419 nicht. Vielmehr werden nur alle bekannten Wirkmechanismen aufgelistet.

Die Herstellung sowohl von funktionellen, dispersen wie auch von vollöslichen Eiweißhydrolysaten wird in der EP 0 480 104 A1 beschrieben. Dabei wird der positive Einfluß eines hohen alkalischen pH-Wertes von 9 bis 11 betont. Besonderer Wert wird darauf gelegt, daß die toxische Aminosäure Lysinoalanin, ein Maß für eine Eiweißschädigung, eine Konzentration von 300 ppm nicht überschreitet. Die Temperaturen bei der enzymatischen Hydrolyse liegen zwischen 10 und 75°C und sind auf die unterschiedlichen verwendeten Proteasen abgestimmt. Die Hydrolysezeit beträgt 10 bis 240 min.

Ein spezielles Verfahren zur Herstellung von Reis-Eiweißhydrolysaten beschreibt die DE 195 02 167 C2. Dabei findet eine Hydrolyse in Gegenwart von Adsorbentien statt. Der pH-Wert liegt zwischen 8 und 10, die Temperatur zwischen 40 und 90°C und die Hydrolysedauer zwischen 1 und 24 Stunden. Durch eine mehrstufige Behandlung der Ausgangssubstanz unter Einbindung von Filtration über Filterpressen und Behandlung mit Aktivkohle ist das bekannte Herstellungsverfahren sehr kostenintensiv. Ein besonderer Nachteil ist auch, daß erst nach einer Reifezeit von 14 Tagen die konservierte Eiweißhydrolysatlösung nach nochmaliger Anwendung von Aktivkohle aufgearbeitet werden kann. Damit ist eine kostengünstige Herstellung von Eiweißhydrolysaten nicht möglich.

Aus der DE 33 06 009 C2 ist es bekannt, zur Hydrolyse von Eiweißen Enzyme auf festen Trägern zu verwenden. Der Schwerpunkt liegt dabei auf Pilzproteasen. Der pH-Bereich, bei dem die Hydrolyse erfolgt, liegt bei 6 bis 9. Die Hydrolysedauer wird über die Strömungsgeschwindigkeit entlang den festen Trägern auf Werte zwischen 10 und 100 min. eingestellt. Letztlich stehen die hohen Kosten der Enzymkopplung an das Trägermaterial, die Entsorgung des Trägermaterials und die Schwierigkeiten bei einer technischen Auslegung einer kommerziellen Umsetzung dieses bekannten Verfahrens entgegen.

Allen oben beschriebenen Verfahren nach dem Stand der Technik, abgesehen von der EP 0 199 981 A2, ist gemeinsam, daß nach der Hydrolyse eine Hitzeinaktivierung der Enzyme sowie eine Abtrennung der nicht hydrolysierten Eiweißbestandteile von den hydrolysierten Eiweißen der Ausgangssubstanz durch Fällung am isoelektrischen Punkt der nicht hydrolysierten Eiweißbestandteile erfolgt, um eine möglichst reine Eiweißhydrolysatlösung zu erhalten.

Unter anderem aus Ullmanns Encyklopädie der technischen Chemie, Band 10, Seite 494 (1975) ist es bekannt, daß die Aktivität von Enzymen in Folge der thermischen, enzymatisch-proteolytischen und mechanischen Belastungen während eines Homogenisiervorgangs, insbesondere durch Hochdruckdesintegration, merklich zurückgebt.

Das der Erfindung am nächsten kommende Verfahren ist aus der WO-A-9 514 394 bekannt. Hier wird eine proteinhaltige Substanz zur Aufbereitung von Proteinen in einem alkalischen Lösungsmittel mit einem pH-Wert > 11,5 und bei einer Temperatur von weniger als 30 °C unter gleichzeitiger Homogenisation dispergiert, und die proteinhaltige Substanz wird vor dem Dispergieren im alkalischen Lösungsmittel mit einer Protease behandelt. Die in Lösung gegangenen Proteine können erneut mit einer weiteren Protease behandelt werden.

Der Erfindung liegt die Aufgabe zugrunde, ein Verfahren zur Herstellung von Eiweißhydrolysaten aus einer Eiweiße in konzentrierter oder isolierter Form enthaltenden Ausgangssubstanz aufzuzeigen, das durch eine kurze Hydrolysezeit in kontinuierlicher und damit wirtschaftlicher Form umsetzbar ist und das bei hoher Flexibilität bezüglich der Ausgangssubstanz und der herzustellenden Eiweißhydrolysate zu einem geringen Bitterstoffgehalt bei dem Verfahrensprodukt führt.

Erfindungsgemäß wird diese Aufgabe durch ein Verfahren mit den Merkmalen des Patentanspruchs 1 gelöst.

Vorteilhafte Ausführungsformen des neuen Verfahrens sind in den Unteransprüchen beschrieben.

Bei dem neuen Verfahren wird die eiweißhaltige Ausgangssubstanz zunächst auf einen pH-Wert von 8 bis 12, vorzugsweise von 9 bis 10, unter Verwendung von Alkali- oder Erdalkalihydroxiden oder -karbonaten eingestellt. Dann wird diese Suspension bei einer Temperatur von unter 80°C, insbesondere bei 40°C, in Gegenwart einer ersten Protease homogenisiert. Die Homogenisation kann mit allen gängigen Homogenisierungstechniken durchgeführt werden. Besonders bevorzugt ist eine Homogenisation durch Hochdruckdesintegration über einen Druckabfall von 50 bis 1000 bar allein oder aber in Kombination mit anderen Homogenisiertechniken. Die erste Protease kann der Suspension unmittelbar vor der Homogenisation oder auch bereits mit gewissem zeitlichem Abstand vorher zugesetzt werden. Nach 1 bis 90 min. nach Beginn des Homogenisierens wird der homogenisierten Dispersion der

Ausgangssubstanz mindestens eine weitere Protease zugesetzt, deren maximale Aktivität in dem voranstehenden pH-Bereich noch mindestens 50 % beträgt. Die Hydrolysezeit unter Homogenisation beträgt nach dem Zusetzen der letzten Protease maximal 60 min., insbesondere 10 bis 15 min., wobei während der Hydrolysezeit vorzugsweise ohne pH-Korrektur gearbeitet wird. Anschließend werden die eingesetzten Enzyme mittels Hitzeeinwirkung bei einem pH-Wert von unter 7,5, insbesondere von unter 6,5 inaktiviert. Für die so durchgeführte enzymatische Hydrolyse eignen sich bis auf saure Proteasen alle anderen Proteasen.

Um lösliche, pH-stabile Eiweißhydrolysate zu erhalten, wird der pH-Wert dann auf den isoelektrischen Punkt der nicht hydrolysierten Eiweiße eingestellt. Dieser liegt insbesondere in dem pH-Bereich von 4,2-4,5. Die sich am isoelektrischen Punkt einstellende Dispersion wird beispielsweise mittels Zentrifugation unter Vakuum oder Membranfiltration bei gleichzeitiger Vermeidung von Schaumbildung in Feststoff und Lösung aufgetrennt. Die gewünschten Eiweißhydrolysate befinden sich in der Lösung. Nach Einstellen eines gewünschten pH-Werts der Lösung kann diese beispielsweise durch Membranfiltration weiter nach Molekulargewichten aufgetrennt werden. Anschließend erfolgt eine Aufkonzentration, in der Regel durch Trocknung.

Als überraschend ist anzusehen, daß trotz der negativen Einflüsse der Homogenisation auf die Aktivität der ersten Protease die Homogenisation den enzymatischen (Teil-)Aufschluß durch die erste Protease in erheblichem Maße fördert, und sich damit das neue Verfahren durch vergleichsweise sehr kurze Prozeßzeiten auszeichnet. Dies mag auf die durch die Homogenisation überproportional vergrößerten Angriffsmöglichkeiten für die enzymatische Spaltung durch die erste Protease zurückzuführen sein. So beschleunigt die enzymatische Hydrolyse unter Homogenisierung der Ausgangssubstanz bei einem pH-Wert größer als 8 die gewünschte Hydrolyse der Proteine ganz erheblich. Dabei kann zunächst mit einer Protease vorgespalten und mit der zweiten Protease nachgespalten werden. Wird auf eine Hochdruckdesintegration als Homogenisiertechnik verzichtet, muß durch eine andere Art der Homogenisation, wie z. B. Ultraschall, Kolloidmühlen, Rotor-Stator-Systeme oder homogenisierende Rührwerke eine intensive Homogenisierung sichtergestellt werden. Empfehlenswert ist in diesem Fall eine Homogeisation über mindestens 15 Minuten bereits vor dem Zufügen der ersten Protease.

Sollten bei dem bis hierher beschriebenen Verfahren in der Hydrolyse der eiweißhaltigen Ausgangssubstanz Bitterstoffe entstehen, so können diese Bitterstoffe reduziert werden, indem eine Zentrifugation unter Vakuum bei einem pH-Wert von etwa 6,5 vor der Hitzeinaktivierung der Proteasen durchgeführt, die erhaltene Lösung aufkonzentriert und anschließend einer Plasteinreaktion unterworfen wird, wobei der pH-Wert mit Alkalioder Erdalkalihydroxiden bei einer Temperatur von unter 35°C auf 11 bis 13 eingestellt wird. Die alkalische Lösung wird dann für 5 bis 30 min. bei dem genannten pH-Wert intensiv gerührt. Dann wird der pH-Wert auf 4,2 bis 7,5 eingestellt, und die Enzyme in der Lösung werden wie vorher beschrieben durch Hitzeeinwirkung inaktiviert, und es erfolgt eine Aufkonzentration der Eiweißhydrolysate durch Trocknung ggf. nach vorheriger Auftrennung nach unterschiedlichen Molekulargewichten.

Die nach dem neuen Verfahren erhaltenen Eiweißhydrolysate weisen je nach Hydrolysegrad eine hohe Funktionalität oder eine volle Löslichkeit auf. Funktionelle Eiweißhydrolysate lassen sich ohne nennenswerte Verluste, lösliche Hydrolysate in Ausbeuten von 60 bis 80 % binnen weniger Minuten herstellen. Dabei ist keine Beschränkung auf einen bestimmten Rohstoff als Ausgangssubstanz gegeben.

Die Erfindung wird im folgenden anhand von Beispielen näher erläutert und beschrieben.

### 1.) Lösliches Soja-Eiweißhydrolysat

1 kg Soja-Eiweißisolat wird mit 6,5 kg 50 °C warmen Wasser versetzt und über 15 Minuten gut homogenisiert. Anschließend wird der pH-Wert mit 25%iger Lösung von Kalziumhydroxid in Wasser auf pH 10.5 angehoben und mit 0,2 Gew.-% einer neutralen Protease versetzt. Dabei bezieht sich die Gewichtsmengenangabe der Protease auf das Gewicht der eingesetzten Eiweiße. Die so erhaltene Dispersion wird 15 min. lang homogenisiert. Die Temperatur der Dispersion steigt dabei leicht an; der pH-Wert sinkt auf 9,5. Unter fortlaufendem Homogenisieren werden nunmehr zunächst 1 bis 1,5 Gew.-% einer alkalischen Protease zugesetzt, wobei sich die Konzentrationsangabe wieder auf das Gewicht der eingesetzten Eiweiße bezieht und wobei die Konzentration auf die jeweilige Ausgangssubstanz so abzustimmen ist, daß der pH-Wert der Dispersion nach etwa 7 min. auf 7 bis 7,5 gefallen ist. Nach den etwa 7 min. wird der pH-Wert mit weiterem Kalziumhydroxid auf 8,2 eingestellt, und eine neutrale Protease wird in einer Konzentration von 1 Gew.-% zugesetzt. Nach 15 min. Homogenisieren beträgt der pH-Wert der Dispersion 7,2. Dann wird die Dispersion mit 15 %iger Schwefelsäure auf pH 6 eingestellt und anschließend für 60 sec. auf 110 °C erhitzt. Nach Abkühlung auf 50°C wird der pH-Wert mit 15%iger Schwefelsäure auf 4,5 eingestellt, und die Dispersion wird unter Vakuum bei 6000 x g zentrifugiert. Der die hydrolysierten Eiweiße enthaltende Überstand kann direkt oder nach Neutralisation sprühgetrocknet werden. Der Überstand ist vollöslich und feststoffrei.

### 2.) Lösliches, klares und bitterfreies Soja-Eiweißhydrolysat

1 kg Soja-Eiweißisolat wird mit 6,5 kg 40°C warmem Wasser versetzt und gut vermischt. Anschließend wird der pH-Wert mit 25%iger Lösung von Natriumhydroxid in Wasser unter gleichzeitiger Zugabe von 0,25 Gew.-% einer alkalischen Protease auf 10,5 angehoben, dabei bezieht sich die Gew.-%-Angabe der alkalischen Protease auf das Gewicht der eingesetzten Eiweiße. Die so erhaltene Dispersion wird 5 min. lang gerührt und dann über einen Druckabfall von 300 bar homogenisiert. Die Temperatur der Dispersion steigt dabei auf 45°C; der pH-Wert sinkt auf 9,0. Anschließend wird unter intensivem Rühren eine zweite alkalische Protease in einer Konzentration von 1,5 Gew.-% bezogen auf die eingesetzten Eiweiße zugesetzt. Die zweite alkalische Protease wird so ausgewählt, daß sie ein gegenüber der ersten Protease unterschiedliches Spaltungsmuster aufweist. Nach 7 min. beträgt der pH-Wert der Dispersion 7 und wird mit weiterem Natriumhydroxid auf 8,2 eingestellt. Anschließend werden 1,5 Gew.-% einer neutralen Protease zugegeben, und es wird für 8 min. weiter hydrolysiert. Dabei fällt der pH-Wert auf 6,5. Die so erhaltene Suspension wird bei 6000 x g zentrifugiert. Der dabei anfallende Überstand wird anschließend für 60 sec. auf 110°C erhitzt und dann im Vakuum auf 30 % Feststoffgehalt aufkonzentriert. Bei Abkühlung auf 30°C werden noch einmal 0,5 Gew.-% der letzten Protease zugegeben, und unter Rühren wird ein pH-Wert von 12 eingestellt. Die resultierende alkalische Lösung wird 15 min. lang intensiv, aber ohne Schaumbildung gerührt. Dann wird mit Salzsäure der pH-Wert der Lösung auf 4,5 eingestellt und die Lösung danach ein zweites Mal für 60 sec. auf 110°C erhitzt. Die hierbei noch ausfallenden nicht hydrolysierten Eiweiße werden bei 50°C und 6000 x g abzentrifugiert und über eine Mikrofiltration mit einer Porengröße von 0,2 µm abfiltriert. Der Überstand bzw. das Permeat kann direkt nach Neutralisation sprühgetrocknet werden. Das gewonnene Eiweißhydrolysat ist vollöslich, klar und bitterstoffrei.

### 3.) Lösliches und klares Weizen-Eiweißhydrolysat ohne Bitterstoffe

1 kg Vital-Gluten wird in 7 kg 40°C warmem Wasser homogenisiert, dem Kalziumhydroxid in einer solchen Menge zugesetzt ist, daß der pH-Wert bei 10,5 liegt. Dieser pH-Wert wird während der Zugabe der eiweißhaltigen Ausgangssubstanz konstant gehalten. Der so erhaltenen Dispersion werden 1,5 Gew.-% einer alkalischen Protease bezogen auf das Gewicht der eingesetzten Eiweiße zugesetzt. Nach 15 min. beträgt der pH-Wert 7,5 und wird mit Kalziumhydroxyd auf 8,2 eingestellt. Anschließend wird eine zweite alkalische Protease, die ein anderes Spaltungsmuster aufweist wie die erste Protease, in einer Konzentration von 1,5 Gew.-% eingesetzt. Nach weiteren 15 min. beträgt der pH-Wert 7,2. Die Dispersion wid mit Salzsäure auf pH 6 gesenkt und bei 6000 x g unter Vakuum zentrifugiert. Die geklärte Flüssigphase wwird erneut mit Salzsäre so versetzt, daß der pH-Wert 4,2 beträgt, und dann für 60 sec. auf 110 °C erhitzt. Nach Abkühlen auf 50 °C wird anschließend nocht einmal unter Vakuum zentrifugiert. Der dabei erhaltene Überstand ist vollöslich, klar und bitterstoffrei. Er kann sofort aufkonzentriert, insbesondere getrocknet werden.

### 4. Lösliches Fischprotein:

1 kg gereinigtes Fischprotein wird in 7 kg 50 °C warmem Wasser aufgenommen, wobei mittels Rotor-Stator-System (Ultraturrax) homogenisiert wird. Hierzu setzt man Natronlauge so zu, daß ein pH-Wert von 11,2 erreicht wird. Unmittelbar nach Erreichen des pH-Wertes werden dieser Dispersion 2 Gew.-% einer alkalischen Protease, bezogen auf den Proteingehalt des Ausgangsmatrials, zugesetzt. Unter starkem Homogenisieren fällt der pH-Wert innerhalb von 10 Minuten auf 9. Ohne die Homogenisation zu unterbrechen, wird eine zweite alkalische Protease zugesetzt, die sich im Spaltungsspektrum von der ersten Protease unterscheidet. Dabei wird die Aktivität so gewählt, daß der pH-Wert innerhalb von 20 Minuten pH 7,2 erreicht.

Diese Dispersion wird bei 6000 x g unter Vakuum zentrifugiert; und die geklärte Phase wird mit Slazsäure auf pH 4,2 eingestellt. Anschließend wird diese Lösung für 60 sec. auf 110 °C erhitzt, sofort anschließend auf 50 °C abgekühlt und erneut unter Vakuum bei 6000 x g zentrifugiert. Die so erhaltene Lösung kann entweder getrocknet oder durch Filtration weiter gereinigt werden.

## Patentansprüche

1. Verfahren zur Herstellung von Eiweißhydrolysaten aus einer Eiweiße in konzentrierter oder isolierter Form enthaltenden Ausgangssubstanz, wobei die Ausgangssubstanz mit einer wässrigen Lösung bei einer Temperatur von 10 bis 80 °C und einem pH-Wert von 8 bis 12 in Gegenwart einer ersten Protease zur enzymatischen Hydrolyse der Eiweiße homogenisiert wird, wobei nach 1 bis 60 Minuten nach Beginn des Homogenisierens zur weiteren enzymatischen Hydrolyse der Eiweiße mindestens eine weitere Protease zugesetzt wird, die sich im Spaltungsspektrum gegenüber der ersten Protease unterscheidet, wobei die Proteasen binnen 1 bis 60 Minuten nach dem Zusetzen der letzten Protease durch Hitzeeinwirkung bei einem pH-Wert kleiner als 7,5 inaktiviert werden, wobei bei einem pH-Wert kleiner als 7,5 ausfallende nicht hydrolysierte Eiweiße abgetrennt werden und wobei die in der Lösung enthaltenen Eiweißhydrolysate aufkonzentriert werden.

2. Verfahren nach Anspruch 1, **dadurch gekennzeichnet, daß** die weitere Protease nach 5 bis 15 Minuten nach Beginn des Homogenisierens zugesetzt wird.

3. Verfahren nach Anspruch 1 oder 2, **dadurch gekennzeichnet, daß** nach 1 bis 60 Minuten nach Beginn des Homogenisierens mindestens zwei weitere Proteasen zugesetzt werden, die sich im Spaltungsspektrum voneinander unterscheiden.

4. Verfahren nach Anspruch 1, 2 oder 3, **dadurch gekennzeichnet, daß** der gesamte Zeitraum vom Beginn des Homogenisierens bis zum Inaktivieren der Proteasen nicht länger als 60 Minuten ist.

5. Verfahren nach einem der Ansprüche 1 bis 4, **dadurch gekennzeichnet, daß** der pH-Wert von 8 bis 12 mit Alkali- oder Erdalkalihydroxiden oder -carbonaten eingestellt wird.

6. Verfahren nach einem der Ansprüche 1 bis 5, **dadurch gekennzeichnet, daß** das Homogenisieren durch einmaliges Hochdruckdesintegrieren des Eiweißisolats in der wässrigen Lösung mit einem Druckabfall von 5 bis 100 Megapascal bewirkt wird.

7. Verfahren nach einem der Ansprüche 1 bis 6, **dadurch gekennzeichnet, daß** die Protease, in deren Gegenwart das Homogenisieren erfolgt, in einer Gewichtskonzentration von weniger als 1 % bezogen auf das Eiweißisolat eingesetzt wird.

8. Verfahren nach einem der Ansprüche 1 bis 7, **dadurch gekennzeichnet, daß** die am isoelektrischen Punkt der nicht hydrolysierten Eiweiße ausfallenden Eiweiße abgetrennt werden.

9. Verfahren nach einem der Ansprüche 1 bis 8, **dadurch gekennzeichnet, daß** der aufkonzentrierten Lösung nochmals eine Protease zugesetzt wird.

10. Verfahren nach einem der Ansprüche 1 bis 9, **dadurch gekennzeichnet, daß** der pH-Wert der aufkonzentrierten Lösung vorübergehend auf 11 bis 13 und ihre Temperatur auf 25 bis 35 °C angehoben wird.

## Claims

1. Method for preparing albumin hydrolysates from a starting material containing albumins in concentrated or isolated form, the starting material being homogenized with an aqueous solution at a temperature of between 10 and 80 °C and at a pH of between 8 and 12 in the presence of a first protease for the enzymatic hydrolysis of the albumins, at least one further protease, the cleaving spectrum of which differs from that of the first protease, being added after between 1 and 60 min. from the start of homogenization for the further enzymatic hydrolysis of the albumins, the proteases being inactivated by heat treatment at a pH below 7,5 within between 1 and 60 minutes from addition of the last protease, the non-hydrolysed albumins precipitated at a pH below 7,5 being separated and the albumin hydrolysates contained in the solution being concentrated.

2. Method according to claim 1, **characterized in that** the further protease is added within between 5 and 15 minutes from the start of homogenization.

3. Method according to claim 1 or 2, **characterized in that** at least two further proteases are added after between 1 and 60 minutes from the start of homogenization, which further proteases differ from each other with regard to their cleaving spectrum.

4. Method according to claim 1, 2 or 3, **characterized in that** the full period from the start of homogenization up to the inactivation of the proteases is no longer than 60 minutes.

5. Method according to any of the claims 1 to 4, **characterized in that** the pH of between 8 and 12 is adjusted with alkaline or alkaline earth hydroxides or carbonates.

6. Method according to any of the claims 1 to 5, **characterized in that** the homogenization is effected by a one time high pressure disintegration of the albumin isolate in an aqueous solution with a drop in pressure of between 5 and 100 MPa.

7. Method according to any of the claims 1 to 6, **characterized in that** the protease which is present during the homogenization is used with a concentration by weight of less than 1 % as related to the albumin isolate.

8. Method according to any of the claims 1 to 7, **characterized in that** the albumins being precipitated at the isoelectric point of the non-hydrolysed albumins are removed.

9. Method according to any of the claims 1 to 8, **characterized in that** a protease is again added to the concentrated solution.

10. Method according to any of the claims 1 to 9, **characterized in that**, for a certain interval of time, the pH of the concentrated solution is increased to between 11 and 13, and its temperature is increased to between 25 and 35 °C.

## Revendications

1. Procédé de production d'hydrolases protéiniques à partir d'une substance de départ contenant des protéines sous forme concentrée ou isolée, la substance de départ étant homogénéisée avec une solution aqueuse à une température de 10 à 80 °C et un pH de 8 à 12, en présence d'une autre protéase pour l'hydrolise enzymatique des protéines, 1 à 60 minutes après le début de l'homogénéisation au moins une autre protéase, qui diffère de la première protéase dans le spectre de scission, étant ajoutée pour la poursuite de l'hydrolise enzymatique des protéines, les protéases étant désactivées, dans un intervalle de 1 à 60 minutes après l'ajout de la dernière protéase, par action de la chaleur avec un pH inférieur à 7,5, les protéines non hydrolisées qui se forment à un pH inférieur à 7,5 étant séparées et les hydrolases protéiniques contenues dans la solution étant concentrées.

2. Pocédé selon la revendication 1, **caractérisé en ce que** l'autre protéase est ajoutée 5 à 15 minutes après le début de l'homogénéisation.

3. Procédé selon la revendication 1 ou 2, **caractérisé en ce qu'**au moins deux autres protéases qui différent l'une de l'autre par le spectre de scission sont ajoutées 1 à 60 minutes après le début de l'homogénéisation.

4. Procédé selon la revendication 1, 2 ou 3, **caractérisé en ce que** toute la durée qui s'écoule depuis le début de l'homogénéisation jusqu'à la désactivation des protéases n'est pas supérieure à 60 minutes.

5. Procédé selon l'une des revendications 1 à 4, **caractérisé en ce que** le pH de 8 à 12 est réglé avec des hydroxides alcalins ou alcalino-terreux.

6. Procédé selon l'une des revendications 1 à 5, **caractérisé en ce que** l'homogénéisation est provoquée par désintégration unique sous haute pression de l'isolat protéinique dans la solution aqueuse avec une chute de pression de 5 à 100 mégapascals.

7. Procédé selon l'une des revendications 1 à 6, **caractérisé en ce que** la protéase, en présence de laquelle s'effectue l'homogénéisation, est introduite dans une concentration en poids inférieure à 1 % par rapport à l'isolat protéinique.

8. Procédé selon l'une des revendications 1 à 7, **caractérisé en ce que** les protéines, qui se produisent au point isoélectrique des protéines non hydrolisées, sont séparées.

9. Procédé selon l'une des revendications 1 à 8, **caractérisé en ce qu'**une protéase est ajoutée encore une fois à la solution concentrée.

10. Procédé selon l'une des revendications 1 à 9, **caractérisé en ce que** le pH de la solution concentrée est relevé provisoirement jusqu'à 11 à 13 et sa température jusqu'à 25 à 35 °C.
